# EUROPEAN PATENT SPECIFICATION

(11) **EP 1 017 388 B1**
(45) Date of publication and mention of the grant of the patent: **20.08.2003**
(21) Application number: 98906926.5
(22) Date of filing: 03.02.1998
(51) Int. Cl.: A61K 31/445, A61P 29/00

(54) **PHARMACEUTICAL COMPOSITIONS CONTAINING IBUPROFEN AND DOMPERIDONE FOR THE TREATMENT OF MIGRAINE**
PHARMAZEUTISCHE ZUBEREITUNGEN ENTHALTEND IBUPROFEN UND DOMPERIDON ZUR BEHANDLUNG VON MIGRÄNE
COMPOSITIONS PHARMACEUTIQUES CONTENANT DE L'IBUPROFENE ET DE LA DOMPERIDONE POUR LE TRAITEMENT DE LA MIGRAINE

(30) Priority: 06.02.1997 GB 9702392
(43) Date of publication of application: 12.07.2000
(73) Proprietor: The Boots Company PLC, Nottingham, Nottinghamshire NG2 3AA (GB)
(72) Inventor: PANKHANIA, Mahendra,Govind, The Boots Company plc, Nottingham NG2 3AA t (GB); YURDAKUL, Saruhan, The Boots Company plc, Nottingham NG2 3AA (GB)
(74) Representative: Frith, Richard William
(86) International application number: EP9800648
(87) International publication number: WO98034612

(56) References cited:
- CA-A- 2 020 018
- GB-A- 2 313 309
- Z.ZAHUMENSZKY ET AL.: "The role of a peripheral dopamine-antagonist (Motilium) in improving the tolerance to steroidal and non-steroidal anti-inflammatory agents" THERAPIA HUNGARICA, vol. 38, no. 4, 1990, pages 156-159, XP002068869
- D.KLINGLER ET AL.: "Migräne-Therapie" OESTERREICHISCHE AERZTEZEITUNG, vol. 49, no. 24, 1994, pages 26-30, XP002068870

## Description

This invention relates to pharmaceutical compositions comprising ibuprofen, and in particular to compositions containing ibuprofen and domperidone useful for the treatment of migraine.

Ibuprofen, namely 2-(4-isobutylphenyl)propionic acid, is a well known medicament with analgesic, anti-inflammatory and anti-pyretic properties. It is usually sold in the form of racemic ibuprofen (equal amounts of the S(+)-ibuprofen and R(-)-ibuprofen enantiomers). It may also be in the form of the purified form of either enantiomer, especially S(+)-ibuprofen which is acknowledged to be the active form of racemic ibuprofen. Ibuprofen is also available in salt form, for example the sodium or lysine salt of ibuprofen. Ibuprofen is available under prescription (eg Brufen (RTM)), primarily for the treatment of painful and anti-inflammatory disorders including rheumatoid arthritis, ankylosing spondylitis, osteoarthritis, postoperative pain, post partum pain and soft tissue injuries, generally at doses up to 3200 mg per day. Ibuprofen is also available as a non-prescription drug (eg Nurofen (RTM)), primarily for the treatment of symptoms of pain and fever including headache, migraine, rheumatic pain, muscular pain, backache, neuralgia, dysmenorrhoea, dental pain and colds and flu, generally at doses up to 1200mg per day. The commercially available ibuprofen tablets usually contain 200mg, 400 mg, 600 mg or 800 mg racemic ibuprofen. When an enantiomer or salt of ibuprofen is used the amount of active substance present may be such that the same therapeutic effect is obtained as from the presently-available doses of racemic ibuprofen. Hereinafter the term "ibuprofen" means any enantiomer of ibuprofen or mixtures of enantiomers including the racemic mixture.

Domperidone, namely 5-chloro-1-[1-[3-(2,3-dihydro-2-oxo-1H-benzimidazol-1-yl)propyl-4-piperidinyl]-1,3-dihydro-2H-benzimidazol-2-one is a well known medicament with antiemetic properties. Domperidone is available under prescription [eg Motilium (RTM)] as tablets for the treatment of functional dyspepsia at doses of up to 80 mg per day or as tablets, suspensions or suppositories for the treatment of emesis (in nausea or vomiting) at oral doses of up to 120 mg per day. Pharmaceutically acceptable salts, eg the maleate salt of domperidone may be used instead of domperidone itself. In this case the amount of active material is adjusted so as to administer an equivalent amount of base.

Patients suffering from migraine experience advantageous relief of the symptoms of migraine when ibuprofen and domperidone are administered simultaneously to the patient according to the present invention.

CA-A-2020018 describes pharmaceutical formulations to treat migraine which contain three essential components, namely an analgesic, an antiemetic and at least one antacid ingredient. The preferred analgesic is acetaminophen, the preferred antiemetic is dimenhydrinate and the preferred antacid ingredient is a mixture of magnesium and aluminium hydroxide. Other analgesics, including ibuprofen are suggested but not exemplified. Other antiemetics, including domperidone are suggested but not exemplified.

It has been proposed that an antiemetic such as metaclopramide or domperidone could be administered to a patient suffering from migraine and that an analgesic such as acetylsalicylic acid (aspirin), paracetamol, naproxen, ibuprofen, diclofenac or metamizole should then be administered after a delay of 10-20 minutes [see Oesterreichische Aerztezeitung 49, 26-30 (1944)].

The present invention therefore provides a pharmaceutical composition comprising a therapeutically effective amount of ibuprofen or a pharmaceutically acceptable salt thereof and a therapeutically effective amount of domperidone or a pharmaceutically acceptable salt thereof but which contains no antacid ingredient. The compositions of the invention can be used to treat migraine. The amount of ibuprofen in the form of its racemate to be administered is generally in the range 200 to 3200 mg per day in one or more doses throughout the day. Preferably each dose contains 50 to 800 (preferably 50 to 400, more preferably 200 to 400) mg of ibuprofen. The amount of domperidone to be administered is generally in the range 10 to 100 (preferably 20 to 100) mg per day in one or more doses throughout the day. Preferably each dose contains 5 to 50 (preferably 5 to 25, more preferably 5 to 20) mg of domperidone. If a pharmaceutically acceptable salt of
either of the active drugs (eg the sodium salt of ibuprofen and/or the maleate salt of domperidone) is used then the amount of drug to be administered should be such that the same therapeutic effect is achieved as from the doses given above. If an enantiomer of ibuprofen (eg S (+) - ibuprofen) is used, the amount of drug should be such that the same therapeutic effect is achieved as from the doses of racemic ibuprofen given above.

The composition comprising a pharmaceutically acceptable diluent or carrier.

The present invention also provides the use of a combination of a therapeutically effective amount of ibuprofen or a pharmaceutically acceptable salt thereof and a therapeutically effective amount of domperidone or a pharmaceutically acceptable salt thereof in the preparation of a medicament for the treatment of migraine which contains no antacid ingredient.

In the following description of suitable dosage forms containing both active drugs the term "active combination" means a combination of ibuprofen or a pharmaceutically acceptable salt thereof and domperidone or a pharmaceutically acceptable salt thereof. The active combination may comprise the two drugs in intimate admixture but may also comprise combinations in which the two drugs are kept apart for example by coating one or both drugs or physically separating the two drugs for example by using a bilayer tablet in which one drug is present in each layer. The active combination may be prepared by suitable mixing prior to adding to other components of the desired dosage forms or it may be prepared in situ during the production of the desired dosage forms by adding the two drugs separately to the other components of the dosage forms. The amounts of each drug in the active combination in each dosage form should be such that the desired total dose of each drug is taken when the patient takes the pharmaceutical compositions of the present invention as directed. Suitably the pharmaceutical compositions are administered in divided doses throughout the day so the amount of ibuprofen (or the corresponding amount of a salt thereof) to be administered at each dosing time is in the range 50 to 800 mg (preferably 50 to 400, more preferably 200 to 400) mg and the amount of domperidone (or the corresponding amount of a salt thereof) to be administered at each dosing time is 5 to 50 (preferably 5 to 25, more preferably 5 to 20) mg. Therefore, if two dosage forms are to be administered at each time the dosage forms should contain 25 to 400 (preferably 25 to 200, more preferably 100 to 200) mg ibuprofen and 2.5 to 25 (preferably 2.5 to 12.5, more preferably 2.5 to 10) mg of domperidone (or the corresponding amounts of their salts).

The pharmaceutical compositions of the present invention may be administered orally, rectally, parenterally, bucally or topically, preferably orally. Suitably the pharmaceutical compositions of the present invention may take the form of any of the known pharmaceutical compositions for oral, rectal, parenteral, buccal or topical administration. Pharmaceutically acceptable diluents or carriers suitable for use in such compositions are well known in the art of pharmacy. The compositions of the invention may contain 1-99% by weight of active combination.

Solid compositions for oral administration are preferred compositions of the invention. Solid compositions of the invention are preferably prepared in unit dosage form and are the known pharmaceutical forms for such administration, for example tablets and capsules. Suitably tablets may be prepared by mixing the active combination with an inert diluent such as calcium phosphate in the presence of disintegrating agents, for example maize starch, and lubricating agents, for example magnesium stearate, and tableting the mixture by known methods. Such tablets may, if desired, be provided with enteric coatings by known methods, for example by the use of cellulose acetate phthalate. Similarly, capsules, for example hard or soft gelatin capsules, containing the active combination optionally in the form of beads with or without added excipients, may be prepared by conventional means and, if desired, provided with enteric coatings in a known manner. The tablets may be formulated in a manner known to those skilled in the art so as to give a controlled release of the compound of the present invention. Other compositions for oral administration include oily suspensions containing a compound of the present invention in a suitable vegetable oil, for example arachis oil or aqueous solutions or suspensions.

Preferably, a solid composition comprises a) 10-99% active combination; b) 1-90% of a diluent and c) 0.1-10% of a lubricating agent, d) 0.1-15% of a disintegrating agent and optionally e) 0.1-15% of a binder. Optionally 0.1-10% of a flow aid may be added.

Suitable diluents include lactose, calcium phosphate, dextrin, microcrystalline cellulose, sucrose, starch, modified starch, calcium sulphate or mixtures thereof. Suitable lubricating agents include magnesium stearate, stearic acid, calcium stearate, sodium stearyl fumarate (sold under the trade name PRUV), hydrogenated vegetable oils or mixtures thereof. Preferably the lubricating agent is magnesium stearate or stearic acid. Suitable disintegrating agents include maize starch, sodium starch glycollate, low substituted hydroxypropyl cellulose, alginic acid, calcium carboxymethyl cellulose, croscarmellose sodium or mixtures thereof. Suitable binders includes polyvinyl pyrrolidone, gelatin, hydroxypropylmethyl cellulose, starch or mixtures thereof. Preferably the binder is polyvinylpyrrolidone. Suitable flow aids include talc and colloidal silicon dioxide. It will be appreciated by those skilled in the art that a particular excipient may perform more than one function for example maize starch may act as a diluent, a binder or as a disintegrating agent.

Controlled release forms of the pharmaceutical compositions of the present invention include rapid release formulations such as soluble granules or melt filled fast release capsules, delayed release formulations such as tablets provided with enteric coatings, for example, of cellulose acetate phthalate and, in particular, sustained release formulations. Numerous types of sustained release formulations are known to those skilled in the art. Typically, the active combination may be encapsulated within a release retarding coating, for example, a copolymer of cellulose ether and acrylate, or may be bound to small particles such as, for example, ion exchange resin beads. Alternatively, the active combination may be incorporated into a matrix containing a release retarding agent such as a hydrophilic gum e.g. xanthan gum, a cellulose derivative eg. hydroxypropyl methylcellulose, or a polysaccharide, wax or plastics material. Such techniques may provide sustained blood levels of ibuprofen and domperidone by controlling, for example, erosion, swelling, disintegration and dissolution of the composition within the gastrointestinal tract.

The active combination may be formulated into a solid dosage form in which the two active drugs are kept separate. For example, the dosage form may be a bilayer tablet in which the active drugs are contained in different layers. The different layers can be formulated so as to provide the optimum release profile for each drug.

Liquid fill compositions for example viscous liquid fills, liquid paste fills or thixotropic liquid fills are also suitable for oral administration. Melt filled compositions may be obtained by mixing the active combination with certain esters of natural vegetable oil fatty acids, for example, the Gelucire (Trademark) range available from Gattefosse to provide a variety of release rates. Suitably a melt-filled capsule comprises a) 10-80% active combination and b) 20-90% of a fatty acid ester excipient which comprises one or more polyol esters and triglycerides of natural vegetable oil fatty acids.

Solid compositions designed to effervesce when added to water to form an effervescent solution or suspension are also suitable for oral administration. Suitably an effervescent composition comprises a) 1-50% of active combination and b) a pharmaceutically acceptable effervescent couple. Such a composition may be presented in the form of tablets or granules. Preferably, effervescent compositions additionally comprise a taste masking component for example a sweetener, a flavouring agent, arginine, sodium carbonate or sodium bicarbonate.

Solid non-effervescent compositions are preferred compositions of the present invention.

Preferably oral liquid compositions comprise a) 0.1-10% active combination possibly as coated particles b) 1-50% of a diluent c) water to 100%. Optionally the composition may contain suspending agents, thickeners, cosolvents such as alcohol and/or preservatives. Suitable diluents include sweetening agents for example sorbitol, xylitol, sucrose, or LYCASIN® (registered trademark of Roquette). Suitable suspending agents or thickeners include cellulose gums, agar or natural gums, for example xanthan gum. Flavourings or other taste-masking agents known to those skilled in the art for example saccharin, sodium saccharin, acesulpham K or aspartame may be added.

Compositions for topical administration may be prepared by dispersing the active combination in a pharmaceutically acceptable cream, ointment or gel. A suitable cream may be prepared by incorporating the active combination in a topical vehicle such as petrolatum and/or light liquid paraffin, dispersed in an aqueous medium using surfactants. A suitable cream comprises a) 1-15% active combination; b) 5-40% of an oily phase; c) 5-15% of an emulsifier; d) 30-85% of water. Suitable oily phases comprise petrolatum and/or light liquid paraffin. Alternatively, the active combination may be distributed in a base comprising a) 10-40% of a self emulsifying base; b) 60-90% of water to form a cream. LABRAFILL and GELOT (both tradenames of Gattefosse) are examples of self emulsifying bases. An ointment may be prepared by mixing the active combination with a topical vehicle such as a mineral oil, petrolatum and/or a wax e.g. paraffin wax or beeswax. A suitable ointment comprises a) 1-15% active combination and b) a topical vehicle to 100%. A gel may be prepared by mixing the active combination with a topical vehicle comprising a gelling agent e.g. basified Carbomer BP, in the presence of water. Suitable gels comprise a) 1-15% active combination; b) 1-20% of a gelling agent; c) 0.01-10% of a preservative and d) water to 100%. Preferably the gelling agent comprises 0.1-10% of a carbomer and a neutralising agent.

Suitable topically administrable compositions may also comprise a matrix in which the active combination is dispersed so that the compounds are held in contact with the skin in order to administer the compounds transdermally, for example in a patch or poultice. A suitable transdermal composition may be prepared by mixing the active combination with a topical vehicle, such as described above, together with a potential transdermal accelerant such as dimethyl sulphoxide, propylene glycol or peppermint oil.

Compositions of the invention suitable for rectal administration are known pharmaceutical forms for such administration, for example suppositories with polyethylene glycol bases or semi-synthetic glycerides. Preferably the composition in the form of a suppository comprises 10-30% active combination and 70-90% of a carrier wherein the carrier is selected from a base which comprises polyethylene glycol or a semi-synthetic glyceride.

Compositions of the invention suitable for parenteral administration are known pharmaceutical forms for such administration, for example sterile suspensions or sterile solutions of the active combination in a suitable solvent.

Spray formulations may be prepared by dissolving or suspending the active combination in a liquid medium which may also contain other ingredients such as stabilising agents, buffering agents, flavourings, sweeteners, colouring agents and preservatives. For example, a spray may be prepared by dissolving water soluble components in water and non-water soluble ingredients in a co-solvent (eg alcohol). The two phases are then mixed and the resulting mixture filtered and placed into dispensing containers. The dispensing containers may be fitted with a metered, manually-operated spray mechanism or the dispenser may contain a pressurised propellant and be fitted with a suitable dispensing valve. The spray and dispensing container may be adapted for nasal administration of the spray

In some formulations it may be beneficial to use the active combination in the form of particles of very small size, for example as obtained by fluid energy milling.

In the compositions of the present invention the components of the active combination may, if desired, be associated with other compatible pharmacologically active ingredients and/or enhancing agents.

The invention will now be illustrated by the following Examples which are given by way of example only.

In these examples the ingredients are obtained from the sources listed below:-
Microcrystalline cellulose is available from FMC Corporation under the trade name Avicel P.H. 101;
Polyvinylpyrrolidone is available from GAF (GB) Limited under the trade name Plasdone K29-32;
Croscarmellose sodium is available from FMC Corporation under the trade name Ac-Di-Sol;
Colloidal silicon dioxide is available from Degussa under the trade name Aerosil 200; and
Calcium carboxymethyl cellulose is available from Carnation Gums, London under the trade name ECG 505.

### Example 1

The example composition comprises the following ingredients:-

| | % w/w |
|---|---|
| Ibuprofen | 57 |
| Domperidone | 2.85 |
| Tricalcium phosphate | 20.15 |
| Microcrystalline cellulose | 6 |
| Polyvinylpyrrolidone (Plasdone) | 3 |
| Croscarmellose sodium | 10 |
| Stearic acid | 1 |

The composition is prepared according to the following stages:-
a) the ibuprofen, domperidone, tricalcium phosphate, croscarmellose sodium and microcrystalline cellulose are sieved and blended to form a homogeneous mixture;
b) the mixture is granulated with polyvinylpyrrolidone and then dried;
c) the dried granules are blended with stearic acid;
d) the lubricated granule is compressed to form tablet cores each containing 200mg of ibuprofen and 10mg of domperidone or each containing 400mg of ibuprofen and 20mg of domperidone; and
e) the tablet cores are coated with a film coating comprising the following ingredients:-

| | % w/w |
|---|---|
| Hydroxypropylmethyl cellulose | 65 |
| Titanium dioxide | 20 |
| French chalk | 15 |

### Example 2

In the same manner as described in Example 1 is prepared the composition of Example 2 comprising the following ingredients in the form of tablets each containing 200 mg of ibuprofen and 5 mg of domperidone or each containing 400 mg of ibuprofen and 10 mg of domperidone.

| | % w/w |
|---|---|
| Ibuprofen | 60 |
| Domperidone | 1.5 |
| Tricalcium phosphate | 18.5 |
| Microcrystalline cellulose | 5.9 |
| Polyvinylpyrrolidone (Plasdone) | 3.9 |
| Croscarmellose sodium | 9.6 |
| Stearic acid | 0.6 |

### Example 3

The example composition comprises the following ingredients:

| | % w/w |
|---|---|
| Ibuprofen | 60.4 |
| Domperidone base (in the form of its maleate salt) | 1.51 |
| Tricalcium phosphate | 18.2 |
| Microcrystalline cellulose | 6 |
| Polyvinylpyrrolidone (Plasdone) | 3.6 |
| Croscarmellose sodium | 9.7 |
| Magnesium stearate | 0.6 |

The composition is prepared according to the following stages:-
(a) the domperidone maleate and microcrystalline cellulose were triturated;
(b) the ibuprofen, tricalcium phosphate, croscarmellose sodium and triturated mixture from (a) are sieved and blended to form a homogeneous mixture;
(c) the mixture is granulated with a solution of polyvinylpyrrolidone in propan-2-ol and then dried;
(d) the dried granules are blended with magnesium stearate;
(e) the lubricated granule is compressed to form tabletcores each containing 200 mg of ibuprofen and 5 mg of domperidone base in the form of its maleate salt or each containing 400 mg of ibuprofen and 10 mg of domperidone in the form of its maleate salt; and
(e) the tablet cores are coated with a film coating comprising the following ingredients:-

| | % w/w |
|---|---|
| Hydroxypropylmethylcellulose | 65 |
| Titanium dioxide | 20 |
| French chalk | 15 |

### Example 4

In a similar manner to that described in Example 3, compositions containing 200 mg of ibuprofen and 2.5 mg of domperidone base (in the form of its maleate salt) or 400 mg of ibuprofen and 5 mg of domperidone base (in the form of its maleate salt) are prepared from the following ingredients:-

| | % w/w |
|---|---|
| Ibuprofen | 60.8 |
| Domperidone base (in the form of its maleate salt) | 0.76 |
| Microcystalline cellulose | 6.1 |
| Tricalcium phosphate | 18.2 |
| Croscarmellose sodium | 9.7 |
| Polyvinylpyrrolidone | 3.6 |
| Magnesium stearate | 0.6 |

### Example 5

In a similar manner to that described in Example 3, compositions containing 200 mg of ibuprofen and 10 mg of domperidone base (in the form of its maleate salt) or 400 mg of ibuprofen and 20 mg of domperidone base (in the form of its maleate salt) are prepared from the following ingredients:-

| | % w/w |
|---|---|
| Ibuprofen | 59.5 |
| Domperidone base (in the form of its maleate salt) | 2.97 |
| Microcystalline cellulose | 5.95 |
| Tricalcium phosphate | 17.9 |
| Croscarmellose sodium | 9.5 |
| Polyvinylpyrrolidone | 3.6 |
| Magnesium stearate | 0.6 |

### Example 6

The composition of Example 6 comprises the following ingredients:-

| | % w/w |
|---|---|
| Ibuprofen | 56 |
| Domperidone | 2.8 |
| Maize starch | 37.2 |
| Dried maize starch | 3.2 |
| Stearic acid | 0.3 |

The composition is prepared according to the following steps:-
(a) the ibuprofen, domperidone and maize starch are sieved and blended to form a homogeneous mixture;
(b) the mixture is granulated with water and allowed to dry;
(c) the dried granules are blended with dried maize starch and stearic acid;
(d) the lubricated granule is compressed to form tablet cores each containing 200 mg of ibuprofen and 10 mg of domperidone or each containing 400 mg of ibuprofen and 20 mg of domperidone;
(e) the tablet cores are coated with a sugar coating comprising the following ingredients:-

| | % w/w |
|---|---|
| Opaglos regular | 1.0 |
| Acacia gum | 1.0 |
| Refined sugar | 73.5 |
| Calcium sulphate | 23.5 |
| Sodium carboxymethyl cellulose | 1.0 |

### Example 7

In the same manner as described in Example 3 the composition of Example 7 is prepared comprising the following ingredients in the form of tablets each containing 200 mg of ibuprofen and 5 mg of domperidone or each containing 400 mg of ibuprofen and 10 mg of domperidone:-

| | % w/w |
|---|---|
| Ibuprofen | 59 |
| Domperidone | 2 |
| Maize starch | 35 |
| Dried maize starch | 3.5 |
| Stearic acid | 0.5 |

### Example 8

The composition of Example 8 comprises the following ingredients:-

| | % w/w |
|---|---|
| Ibuprofen | 70 |
| Domperidone | 3.5 |
| Sodium lauryl sulphate | 0.3 |
| Maize starch | 19.3 |
| Pregelled starch | 5.6 |
| Colloidal silicon dioxide | 0.4 |
| Magnesium stearate | 0.9 |

The example composition is prepared according to the following stages:-
a) the ibuprofen, domperidone, sodium lauryl sulphate, maize starch, pregelled starch, silicone dioxide and magnesium stearate are blended together to form a homogeneous mixture; and
b) the mixture is filled into hard gelatin capsules, each containing 200 mg of ibuprofen and 10mg of domperidone or each containing 400 mg of ibuprofen and 20 mg of domperidone.

### Example 9

In the same manner as described in Example 8, the composition of Example 9 is prepared comprising the following ingredients to give capsules each containing 200mg of ibuprofen and 5 mg domperidone or each containing 400 mg of ibuprofen and 10 mg of domperidone.

| | % w/w |
|---|---|
| Ibuprofen | 75.5 |
| Domperidone | 1.89 |
| Sodium lauryl sulphate | 0.3 |
| Maize starch | 15.41 |
| Pregelled starch | 6 |
| Colloidal silicon dioxide | 0.4 |
| Magnesium stearate | 0.5 |

### Example 10

The composition of Example 10 is prepared comprising the following ingredients:-

| | % w/w |
|---|---|
| Ibuprofen | 61.54 |
| Domperidone | 3.08 |
| Microcrystalline cellulose | 10.22 |
| Croscarmellose sodium | 14.62 |
| Stearic acid | 0.54 |
| Pregelled starch | 10.00 |

The example composition is prepared as follows:
1) The ibuprofen, microcystalline cellulose, 5% of the croscarmellose sodium and the pregelled starch are granulated with water.
2) The granules are dried and sized, blended with the other ingredients and compressed into tablets which are then film coated.

### Example 11

In a similar manner to that described in Example 10, the composition of Example 11 is prepared from the following ingredients to give tablets each containing 200 mg of ibuprofen and 5 mg of domperidone or each containing 400 mg of ibuprofen and 10 mg of domperidone.

| | % w/w |
|---|---|
| Ibuprofen | 61.54 |
| Domperidone | 1.54 |
| Microcrystalline cellulose | 10.89 |
| Croscarmellose sodium | 15.49 |
| Stearic acid | 0.54 |
| Pregelled starch | 10.00 |

### Example 12

The composition of Example 12 is prepared from the following ingredients to give tablets each containing 200 mg of ibuprofen and 5 mg of domperidone or each containing 400 mg of ibuprofen and 10mg of domperidone.

| | % w/w |
|---|---|
| Ibuprofen | 57.14 |
| Domperidone | 1.43 |
| Microcrystalline cellulose | 29.72 |
| Polyvinylpyrrolidone | 0.71 |
| Stearic acid | 0.50 |
| Colloidal silicon dioxide | 0.50 |
| Calcium carboxymethyl cellulose | 10.00 |

The composition is prepared in the following manner
1) The ibuprofen, microcystalline cellulose, part (21%) of the calcium carboxymethyl cellulose and part (50%) of the colloidal silicon dioxide are granulated using a solution of polyvinylpyrrolidone in propan-2-ol.
2) The granules are sized and dried to remove propan-2-ol.
3) The dried granules are blended with the remaining ingredients and compressed into tablets which may be coated if required.

### Example 13

In a similar manner to that described in Example 12 the following ingredients are used to prepare tablets containing 200 mg of ibuprofen and 10 mg of domperidone.

| | % w/w |
|---|---|
| Ibuprofen | 57.14 |
| Domperidone | 2.86 |
| Microcrystalline cellulose | 28.29 |
| Polyvinylpyrrolidone | 0.71 |
| Stearic acid | 0.50 |
| Colloidal silicon dioxide | 0.50 |
| Calcium carboxymethyl cellulose | 10.00 |

### Example 14

A bilayer tablet which contains 200 mg of ibuprofen in a sustained release layer and 10 mg of domperidone in the other layer from which it is released soon after ingestion is prepared from the following ingredients.

| | % w/w |
|---|---|
| Ibuprofen containing layer | |
| Ibuprofen | 77.22 |
| Polyvinylpyrrolidone | 2.51 |
| Stearic acid | 1.00 |
| Xanthan gum | 19 |
| Colloidal silicon dioxide | 0.27 |
| Domperidone containing layer Domperidone | 4.12 |
| Tricalcium phosphate | 56.78 |
| Microcrystalline cellulose | 18.52 |
| Polyvinylpyrrolidone | 4.12 |
| Croscarmellose sodium | 15.64 |
| Magnesium stearate | 0.82 |

The method of making the tablet is as follows:
A) Ibuprofen-containing layer
   The ibuprofen, 3% of the xanthan gum are mixed and granulated using polyvinylpyrrolidone in propan-2-ol. The granules are dried, sized and then blended with the remaining ingredients.
B) Domperidone-containing layer
   The domperidone, tricalcium phosphate, microcrystalline cellulose and croscarmellose sodium are mixed and granulated using polyvinylpyrrolidone in aqueous propan-2-ol. The granules dried, sized and blended with the remaining ingredients.
C) Tablet production
   Using a bilayer tabletting press, the domperidone-containing layer is compressed followed by the ibuprofen-containing layer to give bilayer tablets which may be coated if required

### Example 15

The composition of Example 15 is prepared by mixing the ingredients listed below and preparing tablets each containing 200mg of ibuprofen and 5 mg of domperidone or each containing 400 mg of ibuprofen and 10 mg of domperidone by direct compression.

| | % w/w |
|---|---|
| Ibuprofen | 78.03 |
| Domperidone | 1.95 |
| Microcrystalline cellulose | 10.92 |
| Stearic acid | 1.95 |
| Lactose | 5.19 |
| Sodium lauryl sulphate | 1.05 |
| Colloidal silicon dioxide | 0.12 |
| Magnesium stearate | 0.78 |

### Example 16

In a similar manner to that described in Example 16, directly compressed tablets each containing 200 mg of ibuprofen and 10 mg of domperidone are prepared from the following ingredients:-

| | % w/w |
|---|---|
| Ibuprofen | 78.93 |
| Domperidone | 3.90 |
| Microcrystalline cellulose | 8.97 |
| Stearic acid | 1.95 |
| Lactose | 5.19 |
| Sodium lauryl sulphate | 1.05 |
| Colloidal silicon dioxide | 0.12 |
| Magnesium stearate | 0.78 |

Pharmaceutical compositions of the present invention may also be prepared by replacing the ibuprofen in any one of Examples 1 to 16 by the equivalent amount of an ibuprofen salt (eg the sodium salt) or the activeS(+)-enantiomer thereof and/or by replacing the domperidone by the equivalent amount of a salt of domperidone (eg the maleate salt).

## Claims

1. The use of a combination of a therapeutically effective amount of ibuprofen or a pharmaceutically acceptable salt thereof and a therapeutically effective amount of domperidone or a pharmaceutically acceptable salt thereof in the preparation of a medicament for the treatment of migraine which contains no antacid ingredient.

2. A use as claimed in claim 1 to provide a medicament for the administration of 200-3200 mg of ibuprofen or the corresponding amount of a pharmaceutically acceptable salt or enantiomer thereof and 10-100 mg of domperidone or the corresponding amount of a pharmaceutically acceptable salt thereof per day.

3. A use as claimed in claim 1 or claim 2 to provide a medicament for the administration of 200-3200 mg of ibuprofen or the corresponding amount of a pharmaceutically acceptable salt or enantiomer thereof and 20-100 mg of domperidone or the corresponding amount of a pharmaceutically acceptable salt thereof per day.

4. A use as claimed in any preceding claim to provide a medicament to be administered in more than one dose during the day, each dose comprising 50 to 800 mg of ibuprofen or the corresponding amount of a pharmaceutically acceptable salt or enantiomer thereof and 5 -50 mg of domperidone or the corresponding amount of a pharmaceutically acceptable salt thereof.

5. A use as claimed in any preceding claim to provide a medicament to be administered in more than one dose during the day, each dose comprising 50 to 400 mg of ibuprofen or the corresponding amount of a pharmaceutically acceptable salt or enantiomer thereof and 5 -25 mg of domperidone or the corresponding amount of a pharmaceutically acceptable salt thereof.

6. A use as claimed in any preceding claim to provide a medicament to be administered in more than one dose during the day, each dose comprising 200 to 400 mg of ibuprofen or the corresponding amount of a pharmaceutically acceptable salt or enantiomer and 5 -20 mg of domperidone or the corresponding amount of a pharmaceutically acceptable salt.

7. A use as claimed in claim 1 in which each unit dose of the medicament comprises 25 to 400 mg of ibuprofen or the corresponding amount of a pharmaceutically acceptable salt or enantiomer thereof and 2.5 to 25 mg of domperidone or the corresponding amount of a pharmaceutically acceptable salt thereof.

8. A use as claimed in claim 1 or claim 7 in which each unit dose of the medicament comprises 25 to 200 mg of ibuprofen or the corresponding amount of a pharmaceutically acceptable salt or enantiomer thereof and 2.5 to 12.5 mg of domperidone or the corresponding amount of a pharmaceutically acceptable salt thereof.

9. A use as claimed in any one of claims 1, 7 and 8 in which each unit dose of the medicament comprises 100 to 200 mg of ibuprofen or the corresponding amount of a pharmaceutically acceptable salt or enantiomer thereof and 2.5-10 mg of domperidone or the corresponding amount of a pharmaceutically acceptable salt thereof.

10. A use as claimed in any one of the preceding claims for the preparation of a medicament suitable for oral, rectal, parenteral, buccal or topical administration.

11. A use as claimed in claim 10 for the preparation of a medicament in the form of a tablet, capsule or suppository.

12. A pharmaceutical composition comprising for the treatment of migraine a therapeutically effective amount of ibuprofen or a pharmaceutically acceptable salt thereof and a therapeutically effective amount of domperidone or a pharmaceutically acceptable salt thereof together with a pharmaceutically acceptable diluent or carrier but which contains no antacid ingredient.

13. A pharmaceutical composition as claimed in claim 12 comprising 25 to 400 mg of ibuprofen or the corresponding amount of a pharmaceutically acceptable salt or enantiomer thereof and 2.5 to 25 mg of domperidone or the corresponding amount of a pharmaceutically acceptable salt thereof.

14. A pharmaceutical composition as claimed in claim 12 or claim 13 comprising 25 to 200 mg of ibuprofen or the corresponding amount of a pharmaceutically acceptable salt or enantiomer thereof and 2.5 to 12.5 mg of domperidone or the corresponding amount of a pharmaceutically acceptable salt thereof.

15. A pharmaceutical composition as claimed in any one of claims 12 to 14 comprising 100 to 200 mg of ibuprofen or the corresponding amount of a pharmaceutically acceptable salt or enantiomer thereof and 2.5-10 mg of domperidone or the corresponding amount of a pharmaceutically acceptable salt thereof.

16. A pharmaceutical composition as claimed in any one of claims 12 to 15 which is suitable for oral, rectal, parenteral, buccal or topical administration.

17. A pharmaceutical composition as claimed in claim 16 for the preparation of a medicament in the form of a tablet, capsule or suppository.

## Patentansprüche

1. Verwendung einer Kombination einer therapeutisch wirksamen Menge an Ibuprofen oder eines pharmazeutisch unbedenklichen Salzes davon und einer therapeutisch wirksamen Menge an Domperidon oder eines pharmazeutisch unbedenklichen Salzes davon zur Herstellung eines Antimigränemittels, das keinen antaciden Bestandteil enthält.

2. Verwendung nach Anspruch 1 zur Bereitstellung eines Medikaments zur Verabreichung von täglich 200-3200 mg Ibuprofen bzw. der entsprechenden Menge eines pharmazeutisch unbedenklichen Salzes oder Enantiomers davon und 10-100 mg Domperidon bzw. der entsprechenden Menge eines pharmazeutisch unbedenklichen Salzes davon.

3. Verwendung nach Anspruch 1 oder 2 zur Bereitstellung eines Medikaments zur Verabreichung von täglich 200-3200 mg Ibuprofen bzw. der entsprechenden Menge eines pharmazeutisch unbedenklichen Salzes oder Enantiomers davon und 20-100 mg Domperidon bzw. der entsprechenden Menge eines pharmazeutisch unbedenklichen Salzes davon.

4. Verwendung nach einem der vorhergehenden Ansprüche zur Bereitstellung eines Medikaments, das über den Tag in mehr als einer Dosis verabreicht wird, wobei jede Dosis 50 bis 800 mg Ibuprofen bzw. die entsprechende Menge eines pharmazeutisch unbedenklichen Salzes oder Enantiomers davon und 5-50 mg Domperidon bzw. die entsprechende Menge eines pharmazeutisch unbedenklichen Salzes davon enthält.

5. Verwendung nach einem der vorhergehenden Ansprüche zur Bereitstellung eines Medikaments, das über den Tag in mehr als einer Dosis verabreicht wird, wobei jede Dosis 50 bis 400 mg Ibuprofen bzw. die entsprechende Menge eines pharmazeutisch unbedenklichen Salzes oder Enantiomers davon und 5-25 mg Domperidon bzw. die entsprechende Menge eines pharmazeutisch unbedenklichen Salzes davon enthält.

6. Verwendung nach einem der vorhergehenden Ansprüche zur Bereitstellung eines Medikaments, das über den Tag in mehr als einer Dosis verabreicht wird, wobei jede Dosis 200 bis 400 mg Ibuprofen bzw. die entsprechende Menge eines pharmazeutisch unbedenklichen Salzes oder Enantiomers und 5-20 mg Domperidon bzw. die entsprechende Menge eines pharmazeutisch unbedenklichen Salzes enthält.

7. Verwendung nach Anspruch 1, wobei jede Einheitsdosis des Medikaments 25 bis 400 mg Ibuprofen bzw. die entsprechende Menge eines pharmazeutisch unbedenklichen Salzes oder Enantiomers davon und 2,5 bis 25 mg Domperidon bzw. die entsprechende Menge eines pharmazeutisch unbedenklichen Salzes davon enthält.

8. Verwendung nach Anspruch 1 oder Anspruch 7, wobei jede Einheitsdosis des Medikaments 25 bis 200 mg Ibuprofen bzw. die entsprechende Menge eines pharmazeutisch unbedenklichen Salzes oder Enantiomers davon und 2,5 bis 12,5 mg Domperidon bzw. die entsprechende Menge eines pharmazeutisch unbedenklichen Salzes davon enthält.

9. Verwendung nach einem der Ansprüche 1, 7 und 8, wobei jede Einheitsdosis des Medikaments 100 bis 200 mg Ibuprofen bzw. die entsprechende Menge eines pharmazeutisch unbedenklichen Salzes oder Enantiomers davon und 2,5 bis 10 mg Domperidon bzw. die entsprechende Menge eines pharmazeutisch unbedenklichen Salzes davon enthält.

10. Verwendung nach einem der vorhergehenden Ansprüche zur Herstellung eines Medikaments, das für die orale, rektale, parenterale, bukkale oder topische Verabreichung geeignet ist.

11. Verwendung nach Anspruch 10 zur Herstellung eines Medikaments in Form einer Tablette, einer Kapsel oder eines Zäpfchens.

12. Pharmazeutische Zusammensetzung zur Behandlung von Migräne, enthaltend eine therapeutisch wirksame Menge an Ibuprofen oder eines pharmazeutisch unbedenklichen Salzes davon und eine therapeutisch wirksame Menge an Domperidon oder eines pharmazeutisch unbedenklichen Salzes davon zusammen mit einem pharmazeutisch unbedenklichen Verdünnungsmittel oder Trägerstoffs, die jedoch keinen antaciden Bestandteil enthält.

13. Pharmazeutische Zusammensetzung nach Anspruch 12, enthaltend 25 bis 400 mg Ibuprofen bzw. die entsprechende Menge eines pharmazeutisch unbedenklichen Salzes oder Enantiomers davon und 2,5 bis 25 mg Domperidon bzw. die entsprechende Menge eines pharmazeutisch unbedenklichen Salzes davon.

14. Pharmazeutische Zusammensetzung nach Anspruch 12 oder Anspruch 13, enthaltend 25 bis 200 mg Ibuprofen bzw. die entsprechende Menge eines pharmazeutisch unbedenklichen Salzes oder Enantiomers davon und 2,5 bis 12,5 mg Domperidon bzw. die entsprechende Menge eines pharmazeutisch unbedenklichen Salzes davon.

15. Pharmazeutische Zusammensetzung nach einem der Ansprüche 12 bis 14, enthaltend 100 bis 200 mg Ibuprofen bzw. die entsprechende Menge eines pharmazeutisch unbedenklichen Salzes oder Enantiomers davon und 2,5 bis 10 mg Domperidon bzw. die entsprechende Menge eines pharmazeutisch unbedenklichen Salzes davon.

16. Pharmazeutische Zusammensetzung nach einem der Ansprüche 12 bis 15, die sich zur oralen, rektalen, parenteralen, bukkalen oder topischen Verabreichung eignet.

17. Pharmazeutische Zusammensetzung nach Anspruch 16, die sich zur Herstellung eines Medikaments in Form einer Tablette, einer Kapsel oder eines Zäpfchens eignet.

## Revendications

1. Utilisation d'une combinaison d'une quantité thérapeutiquement efficace d'ibuprofène ou d'un sel pharmaceutiquement acceptable de celui-ci et d'une quantité thérapeutiquement efficace de dompéridone ou d'un sel pharmaceutiquement acceptable de celle-ci dans la préparation de médicament destiné au traitement de la migraine ne contenant aucun ingrédient anti-acide.

2. Utilisation selon la revendication 1, pour fournir un médicament destiné à l'administration de 200-3200 mg d'ibuprofène ou de la quantité correspondante d'un sel ou d'un énantiomère pharmaceutiquement acceptable de celui-ci et de 10-100 mg de dompéridone ou de la quantité correspondante d'un sel pharmaceutiquement acceptable de celle-ci par jour.

3. Utilisation selon la revendication 1 ou la revendication 2, pour fournir un médicament destiné à l'administration de 200-3200 mg d'ibuprofène ou de la quantité correspondante d'un sel ou d'un énantiomère pharmaceutiquement acceptable de celui-ci et de 20-100 mg de dompéridone ou de la quantité correspondante d'un sel pharmaceutiquement acceptable de celle-ci par jour.

4. Utilisation selon l'une quelconque des revendications précédentes, pour fournir un médicament destiné à être administré en plus d'une dose par jour, chaque dose comprenant 50 à 800 mg d'ibuprofène ou de la quantité correspondante d'un sel ou d'un énantiomère pharmaceutiquement acceptable de celui-ci et de 5-50 mg de dompéridone ou de la quantité correspondante d'un sel pharmaceutiquement acceptable de celle-ci.

5. Utilisation selon l'une quelconque des revendications précédentes, pour fournir un médicament destiné à être administré en plus d'une dose par jour, chaque dose comprenant 50 à 400 mg d'ibuprofène ou de la quantité correspondante d'un sel ou d'un énantiomère pharmaceutiquement acceptable de celui-ci et de 5-25 mg de dompéridone ou de la quantité correspondante d'un sel pharmaceutiquement acceptable de celle-ci.

6. Utilisation selon l'une quelconque des revendications précédentes, pour fournir un médicament destiné à être administré en plus d'une dose par jour, chaque dose comprenant 200 à 400 mg d'ibuprofène ou de la quantité correspondante d'un sel ou d'un énantiomère pharmaceutiquement acceptable de celui-ci et de 5-20 mg de dompéridone ou de la quantité correspondante d'un sel pharmaceutiquement acceptable de celle-ci.

7. Utilisation selon la revendication 1, dans laquelle chaque dose unitaire du médicament comprend 25 à 400 mg d'ibuprofène ou de la quantité correspondante d'un sel ou d'un énantiomère pharmaceutiquement acceptable de celui-ci et 2,5 à 25 mg de dompéridone ou de la quantité correspondante d'un sel pharmaceutiquement acceptable de celle-ci.

8. Utilisation selon la revendication 1 ou la revendication 7, dans laquelle chaque dose unitaire du médicament comprend 25 à 200 mg d'ibuprofène ou de la quantité correspondante d'un sel ou d'un énantiomère pharmaceutiquement acceptable de celui-ci et 2,5 à 12,5 mg de dompéridone ou de la quantité correspondante d'un sel pharmaceutiquement acceptable de celle-ci.

9. Utilisation selon l'une quelconque des revendications 1, 7 et 8, dans laquelle chaque dose unitaire du médicament comprend 100 à 200 mg d'ibuprofène ou de la quantité correspondante d'un sel ou d'un énantiomère pharmaceutiquement acceptable de celui-ci et 2,5 à 10 mg de dompéridone ou de la quantité correspondante d'un sel pharmaceutiquement acceptable de celle-ci.

10. Utilisation selon l'une quelconque des revendications précédentes, pour la préparation d'un médicament adapté à l'administration par voie orale, rectale, parentérale, buccale ou topique.

11. Utilisation selon la revendication 10, pour la préparation d'un médicament sous forme de comprimé, de capsule ou de suppositoire.

12. Composition pharmaceutique comprenant, pour le traitement de la migraine, une quantité thérapeutiquement efficace d'ibuprofène ou d'un sel pharmaceutiquement acceptable de celui-ci et une quantité thérapeutiquement efficace de dompéridone ou d'un sel pharmaceutiquement acceptable de celle-ci conjointement avec un diluant ou un support pharmaceutiquement acceptable mais ne contenant aucun ingrédient anti-acide.

13. Composition pharmaceutique selon la revendication 12, comprenant 25 à 400 mg d'ibuprofène ou la quantité correspondante d'un sel ou d'un énantiomère pharmaceutiquement acceptable de celui-ci et 2,5 à 25 mg de dompéridone ou de la quantité correspondante d'un sel pharmaceutiquement acceptable de celle-ci.

14. Composition pharmaceutique selon la revendication 12 ou la revendication 13, comprenant 25 à 200 mg d'ibuprofène ou la quantité correspondante d'un sel ou d'un énantiomère pharmaceutiquement acceptable de celui-ci et 2,5 à 12,5 mg de dompéridone ou de la quantité correspondante d'un sel pharmaceutiquement acceptable de celle-ci.

15. Composition pharmaceutique selon l'une quelconque des revendications 12 à 14, comprenant 100 à 200 mg d'ibuprofène ou la quantité correspondante d'un sel ou d'un énantiomère pharmaceutiquement acceptable de celui-ci et 2,5-10 mg de dompéridone ou de la quantité correspondante d'un sel pharmaceutiquement acceptable de celle-ci.

16. Composition pharmaceutique selon l'une quelconque des revendications 12 à 15, qui est adaptée à l'administration par voie orale, rectale, parentérale, buccale ou topique.

17. Composition pharmaceutique selon la revendication 16, pour la préparation d'un médicament sous forme de comprimé, de capsule ou de suppositoire.
